(19) Europäisches Patentamt / European Patent Office / Office européen des brevets

(11) **EP 3 542 839 B1**

(12) **EUROPEAN PATENT SPECIFICATION**

(45) Date of publication and mention of the grant of the patent:
**23.10.2024 Bulletin 2024/43**

(21) Application number: **19173852.5**

(22) Date of filing: **07.02.2018**

(51) International Patent Classification (IPC):
***A61M 1/16*** *(2006.01)*

(52) Cooperative Patent Classification (CPC):
**A61M 1/1613; A61M 1/1605; A61M 1/1696;**
A61M 2205/3334; A61M 2205/3379

(54) **PERSONALIZED BICARBONATE MANAGEMENT SYSTEMS AND METHODS FOR SORBENT-BASED HEMODIALYSIS**

PERSONALISIERTE BIKARBONATMANAGEMENTSYSTEME FÜR SORPTION BASIERTE HÄMODIALYSE

SYSTÈMES DE GESTION DE BICARBONATE PERSONNALISÉE POUR DIALYSE À BASE DE SORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2017 US 201762456700 P**
**24.03.2017 NL 2018577**
**16.01.2018 US 201815872363**

(43) Date of publication of application:
**25.09.2019 Bulletin 2019/39**

(62) Document number(s) of the earlier application(s) in accordance with Art. 76 EPC:
**18155635.8 / 3 360 587**

(73) Proprietor: **Mozarc Medical US LLC**
**Minneapolis, MN 55431 (US)**

(72) Inventors:
- **MAZACK, Michael J.M.**
**Minneapolis, MN 55432 (US)**
- **GROVENDER, Eric A.**
**Minneapolis, MN 55432 (US)**
- **PUDIL, Bryant**
**Minneapolis, MN 55432 (US)**

(74) Representative: **Maschio & Soames IP Ltd**
**30 Carlton Crescent**
**Southampton SO15 2EW (GB)**

(56) References cited:
**US-A1- 2013 213 890      US-A1- 2014 158 588**
**US-A1- 2015 343 126**

**Description**

FIELD OF INVENTION

The present disclosure relates to devices, systems, and methods for managing base levels, specifically bicarbonate, in an individual undergoing dialysis. The devices, systems, and methods can be compatible with a sorbent-based dialysis therapy, and open loop control of base levels in the dialysis, without sensors or feedback for controlling the introduction of bicarbonate into the dialysate. The bicarbonate levels can be controlled and personalized to a patient using certain predetermined patient parameters. Related systems, algorithms, and control systems are contemplated including the specific predetermined parameters predictive of post-dialysis bicarbonate levels.

BACKGROUND

The dominant pH buffer in the human body is the bicarbonate-dissolved $CO_2$ (or carbonic acid) buffer system. Chronic renal failure causes metabolic acidosis in most patients. In metabolic acidosis, the pH of a patient's blood is low because the concentration of bicarbonate in their blood is below normal. One of the most important outcomes of renal replacement therapy is the effective management of patient acid-base status through controlling the concentration of bicarbonate and bicarbonate predecessors in the dialysate bath.

Total, pre-dialysis, bicarbonate levels in a patient can be predictive of patient survival. Patient post-dialysis bicarbonate levels may also be predictive of pre-dialysis levels, and therefore patient survival. Managing patient acid-base homeostasis whether bicarbonate or acetate during and after therapy can be important functions of dialysis therapy. However, a post-hemodialysis (HD) total base level of bicarbonate is not generally clinically targeted, and the post-hemodialysis (HD) total base level is not generally used to determine the pre-therapy target blood bicarbonate level for a next therapy session. In a single-pass hemodialysis system, the bicarbonate concentration entering the dialyzer is generally constant with spent dialysate being discarded after passing through the dialyzer a single time. However, the dialysate used in single-pass systems cannot be easily adjusted to deliver a desired amount of base to the patient. In sorbent-based systems, dialysate is recirculated with a portion of the spent dialysate being regenerated before reentering the dialyzer. However, the final bicarbonate concentration of the dialysate entering the dialyzer can vary widely from patient to patient because the bicarbonate concentration depends on many factors such as patient parameters, features of the sorbent-based dialysis system, and related system parameters. Patients being dialyzed on the same machine using the same settings could have very different blood bicarbonate levels at the end of therapy. Although certain closed-loop control systems have attempted to monitor bicarbonate levels and then make adjustments by adding varying levels of bicarbonate, the systems require one or more sensors to monitor the dialysate bicarbonate levels. The sensors and related control circuitry increase the computational and mechanical complexity of the dialysis system and drive up manufacturing and operating costs. The increased complexity also increases the possibility of malfunction. Known systems also fail to account for the physiological parameters of individual patients, such as acetate metabolism and other similar health-related parameters that are important in maintaining a desired post-HD bicarbonate level.

There is a need for systems and methods for controlling and managing a post-hemodialysis (HD) bicarbonate or acid/base level that can be clinically targeted. There is also a need for controlling the addition of bicarbonate that targets a particular patient, which can include control over a base pump flow rate that adds a base or base equivalent such as a bicarbonate buffer solution to result in a desired post-therapy bicarbonate level. The related systems and methods should be compatible with a sorbent-based hemodialysis system and able to achieve a desired therapy bicarbonate and/or post-therapy bicarbonate level in the patient. To obtain the desired therapy goals for sorbent based hemodialysis, there is a need for related algorithms, controllers, computers, computer-based methods, algorithms, control logic, processors, software, and logic for developing and implementing personalized control algorithms to predict and control the final base level in a patient, post-dialysis. Ideally, the systems and methods should not solely need to rely on sensors for bicarbonate monitoring. The systems and methods should be compatible with sorbent based dialysis.

SUMMARY OF THE INVENTION

A dialysis system according to the present invention comprises the technical features as defined in independent claim 1.

The first aspect of the present disclosure is drawn to a system. The system includes a dialysate flow path, the dialysate flow path having a dialyzer, dialysate pump, and a bicarbonate source; and a processor programmed to obtain one or more patient parameters and one or more system parameters and to set a flow rate or profile of bicarbonate from the bicarbonate source to the dialysate flow path, wherein the processor sets the flow rate of bicarbonate from the bicarbonate source to the dialysate flow path based on a user specified patient post-dialysis dialysate bicarbonate level..The dialysis parameters can include one or more of: dialysis treatment duration, ultrafiltration rate and/or ultrafiltration profile, dialysate flow rate and/or profile, blood flow rate and/or profile, sodium prescription and/or profile, infusate prescription and/or profile, sorbent cartridge mass, sorbent cartridge layer order, sorbent cartridge pH and/or profile, dialyzer efficiency, degasser outlet $PCO_2$ levels and/or profile, whether bicarbonate is to be metered by a flow-through cartridge or liquid concentrate, dialysis mode, and dialysate temperature.

The patient parameters can include one or more of: patient pre-dialysis bicarbonate level, patient pre-dialysis urea level, patient pre-dialysis weight, patient acid generation, and patient bicarbonate consumption rate.

The processor can set a dialysate bicarbonate prescription or profile based on the user specified patient post-dialysis bicarbonate level by an iterative algorithm.

The processor can be programmed to operate a mathematical model using the one or more patient parameters and the one or more dialysis parameters to set a personalized bicarbonate addition profile and a set patient bicarbonate concentration.

The processor can be programmed to determine whether a difference between the user specified patient bicarbonate level and the set patient bicarbonate concentration is greater than a predetermined threshold.

At least one patient parameter can be obtained from population averages and/or historical values.

The dialysate flow path includes a dialysate regeneration module.

The dialysate regeneration module contains urease.

The dialysate regeneration module modulates an amount of bicarbonate in a dialysate. The processor can be programmed to set the flow rate of bicarbonate using any of: a) curve fitting; b) partial differential equations; c) ordinary differential equations; or b) a lookup table.

The curve fitting can include a) a linear transfer function; or b) non-linear transfer function.

The processor can be programmed to set a bicarbonate addition profile, wherein the bicarbonate addition profile uses steps, linear interpolations, non-linear interpolations, and/or continuous addition.

The processor can be programmed to set a second flow rate of bicarbonate during dialysis treatment.

The second set flow rate can be based upon an update of one or more patient or system parameters.

The second set flow rate of bicarbonate can be based on clearance, pH, and/or conductivity of the dialysate.

The system can include a conductivity sensor in the dialysate flow path, and the processor can be programmed to control the flow rate of bicarbonate based on a conductivity of the dialysate.

The system of the present disclosure can be used in a method of controlling bicarbonate addition to a dialysate flow path, which is not part of the present invention. The method (not claimed) includes the steps of a) obtaining one or more patient parameters and one or more system parameters; b) setting a flow rate of bicarbonate from a bicarbonate source to the dialysate flow path; and c) controlling addition from the bicarbonate source to the dialysate flow path to result in a user specified patient post-dialysis bicarbonate level.

The method can be performed by a dialysis system.

The dialysis parameters can include one or more of dialysis treatment duration, ultrafiltration rate and/or profile, dialysate flow rate and/or profile, blood flow rate and/or profile, sodium prescription and/or profile, infusate prescription and/or profile, sorbent cartridge mass, sorbent cartridge layer order, sorbent cartridge pH and/or profile, dialyzer efficiency, degasser outlet $PCO_2$ levels and/or profile, whether bicarbonate is to be metered by a flow-through cartridge or liquid concentrate, dialysis mode, and dialysate temperature.

The patient parameters can include one or more of patient pre-dialysis bicarbonate level, patient pre-dialysis urea level, patient pre-dialysis weight, patient acid generation, and patient bicarbonate consumption rate.

Also disclosed is identifying significant parameters for predicting a patient post-dialysis bicarbonate level from one or more patient parameters obtained from a patient and one or more system parameters obtained from a system; generating one or more control algorithms for setting a flow rate of bicarbonate based on the significant parameters using curve fitting for determining the flow rate of bicarbonate; and applying the control algorithms by inputting data for the one or more patient parameters, wherein the flow rate of bicarbonate is determined from the control algorithms to cause the patient post-dialysis bicarbonate level.

The step of generating the control algorithms can include curve fitting for predicting the patient post-dialysis bicarbonate level, the curve fitting can include the significant parameters as any of main effects or two-way interactions.

The step of generating the control algorithms can include curve fitting of one or more equations as a predictor for the flow rate of bicarbonate, and curve fitting of one or more equations as a corrector based on additional data of the significant parameters.

The prior art includes US 2015/343126 A1, which discloses a method for treating dialysate, a dialysis system and a method for pre-evaluating dialysis patients for treatment.

However, this document does not teach a system that uses a dialysate precursor having bicarbonate.

US 2015/343126 A1 discloses that spent dialysate is passed through a sorbent cartridge, generating bicarbonate. Bicarbonate is then added to the dialysate early in treatment to generate a dialysate bicarbonate concentration within a specified range, and the bicarbonate addition is stopped at some point where the generated bicarbonate from the sorbent cartridge reaches the specified dialysate bicarbonate level.

The system and method disclosed in US 2015/343126 A1 are limited to controlling the amount of bicarbonate in the dialysate after passage through the sorbent cartridge.

The present invention, in contrast, allows for control over the patient's post-dialysis bicarbonate level.

US 2015/343126 A1 does not disclose or hint at that the patient post-dialysis bicarbonate level can be controlled. Rather US 2015/343126 A1 is limited to controlling the post-sorbent dialysate bicarbonate level and provides no teaching or suggestion on how the patient and system input parameters could be used to calculate and determine a patient post-dialysis bicarbonate level.

The prior art also includes US 2013/213890 A1 which discloses a modular hemodialysis system.

This document teaches using a bicarbonate cartridge through which dialysate is diverted to add bicarbonate to the dialysate. In certain embodiments, the amount of urea absorbed by the sorbent cartridge can be calculated, and based on this calculation an amount of bicarbonate can be added to the dialysis circuit to maintain a specified dialysate pH. However, bicarbonate addition in US 2013/213890 A1 s based on maintaining the dialysate pH.

There is no teaching that the bicarbonate addition can be controlled based on a user specified patient post-dialysis bicarbonate level

The prior art also includes US 2014/158588 A1, which discloses a pH and buffer management system for hemodialysis systems.

This document teaches adjusting the dialysate pH or buffer concentration to generate a predetermined total bicarbonate concentration in the dialysate, and not to control the patient bicarbonate level to a user specified patient post-dialysis bicarbonate level. Rather, US 2014/158588 A1 teaches that a dialysate bicarbonate concentration is determined, and a reconstitution system is used to rebalance the dialysate for electrolytes and buffer.

BRIEF DESCRIPTION OF THE DRAWINGS

FIG. 1 shows a sorbent-based hemodialysis apparatus including a controller, a blood circuit, a dialysis circuit, and a liquid concentrate infusate system.

FIG. 2 shows a sorbent-based hemodialysis apparatus including a controller, a blood circuit, a dialysis circuit, and a flow-through bicarbonate cartridge.

FIG. 3 shows an algorithm for controlling bicarbonate addition to a dialysate flow path.

FIG. 4 shows examples of bicarbonate concentrate delivery throughout therapy.

FIG. 5 shows examples of bicarbonate concentrate delivery in an interval between discrete time points.

FIG. 6 is a flow chart for determining variables significant to patient post-dialysis bicarbonate levels.

DETAILED DESCRIPTION OF THE INVENTION

Unless defined otherwise, all technical and scientific terms used have the same meaning as commonly understood by one of ordinary skill in the art.

The articles "a" and "an" are used to refer to one or to more than one (*i.e.*, to at least one) of the grammatical object of the article. For example, "an element" means one element or more than one element.

The terms "applying," or to "apply" can refer to bringing into action a particular event or instruction. For example, an algorithm can be applied to a particular method by using an algorithm with initial parameters to determine a state of a system or a patient.

The term "bicarbonate source" can refer to a source of bicarbonate ions in solid and/or solution form. The bicarbonate ions can be present as a bicarbonate salt of any type. The bicarbonate source can contain at least one fluid pathway and include components such as conduits, valves, filters or fluid connection ports, any of which are fluidly connectable to each other or to a fluid flow path. The bicarbonate source can either be formed as a stand-alone enclosure or a compartment integrally formed with an apparatus containing the bicarbonate source.

The term "bicarbonate" can refer to either bicarbonate anions or predecessors to bicarbonate anions, such as salts of bicarbonate, e.g. sodium bicarbonate. Predecessors to bicarbonate anions are any substances that can convert to bicarbonate anions based on conditions within a system or within a patient, such as acetate, lactate, and/or citrate.

The term "blood flow profile" can refer to a set blood flow rates used to describe the flow of blood at any point of time. For example, the blood flow profile can describe flow during a therapy.

"Blood flow rate" can refer to the rate at which blood is flowed through a system. For example, the blood flow rate can describe the rate of blood flow through a dialyzer during therapy.

The phrase "to cause," "causing," and the like refer to the act of giving rise or resulting in a specified condition or state.

The term "clearance" can refer to a measurement of the volume of plasma from which a substance is removed per unit time.

The term "comprising" includes, but is not limited to, whatever follows the word "comprising." Use of the term indicates the listed elements are required or mandatory but that other elements are optional and may be present.

The term "conductivity of a dialysate" can refer to the inverse of the electrical resistance of the dialysate.

The term "conductivity sensor" can refer to any component capable of measuring the electrical conductance or the electrical resistance of a fluid.

The term "consisting essentially of" includes whatever follows the term "consisting essentially of" and additional elements, structures, acts or features that do not affect the basic operation of the apparatus, structure or method described.

The term "consisting of" includes and is limited to whatever follows the phrase "consisting of." The phrase indicates the limited elements are required or mandatory and that no other elements may be present.

The term "continuous addition" refers to an uninterrupted or nearly uninterrupted addition of a substance at a rate that can be constant or variable over a desired period of time. Continuous addition is not limited to any particular rate of addition and, for example, can occur by adding a substance at first rate for a first period of time, continue at a second rate for a second period of time, and end at a third rate for a third period of time. Linear and non-linear rates of addition are contemplated. The continuous addition can be an instantaneous flow rate that is a function of time such as a line or a curve. Moreover, continuous addition can occur by a step function or by repeated short pulses at a frequency sufficient to be effectively continuous for the addition of that particular substance.

A "control algorithm" can refer to a type of algorithm that describes the nature of a control system or implements a set of instructions. A control system can apply control algorithms to variables against a set point, and generate an output per the control algorithms.

The term "controlling addition" refers to operation of pumps or valves to accurately control the amount of a substance flowed into a dialysate flow path.

A "corrector" can refer to a function that refines an initial approximation from a predictor function to a more accurate result.

The term "curve fitting" can refer to generating linear and/or non-linear transfer functions, lookup tables, fitting data from design of experiments, and regression analysis including but not limited to linear regression, Bayesian linear regression, neural network regression, decision forest regression, logistic regression, and boosted decision tree regression.

A "degasser" is a component capable of removing dissolved and undissolved gasses from fluids.

The term "degasser outlet $PCO_2$ levels" can refer to the partial pressure of carbon dioxide at an outlet of a degasser.

A "degasser outlet $PCO_2$ profile" can refer to a variable $PCO_2$ level as a function of time and/or volumetric flow.

The terms "determining" and "determine" can refer to ascertaining or identifying a particular state or desired state. As used in "determining significant parameters," the phrase refers to ascertaining or identifying any parameter. For example, a system or fluid, or any measured variable(s) or feature(s) of a system or a fluid can be determined by obtaining sensor data, retrieving data, performing a calculation, or by any other known method.

The term "dialysate" describes a fluid into or out of which solutes from a fluid to be dialyzed diffuse through a membrane. A dialysate typically contains electrolytes close in concentration to the physiological concentration of electrolytes found in blood.

"Dialysate bicarbonate concentration" can refer to the concentration of bicarbonate ions in a dialysate.

The term "dialysate flow path" can refer to a fluid pathway or passageway that conveys a fluid, such as dialysate and is configured to form at least part of a fluid circuit for peritoneal dialysis, hemodialysis, hemofiltration, hemodiafiltration or ultrafiltration.

The term "dialysate flow profile" can refer to one or more specified dialysate flow rates and/or lengths of time for performing or halting ultrafiltration to result in a description of the dialysate flow. The profile can include any one or more of plural rates, start/stop instructions, and length of time to be used during therapy, wherein any can vary as a function of time. The profile can also refer to a constant or variable rate of dialysate flow specific to a particular patient or subject over time and/or volumetric flow. The variable rate can include an instantaneous flow rate that is a function of time and/or volumetric flow, such as a curve or a line, or a set period of time. The profile can include a period of time wherein no addition of dialysate flow is required.

"Dialysate flow rate" can refer to the rate at which dialysate is pumped through a dialysate flow path.

The term "dialysate pump" can refer to any device that causes the movement of fluids or gases by applying negative or positive pressure.

The "dialysate regeneration module" can refer to a system for removing certain electrolytes and waste species, such as urea, from a dialysate after contact with a dialyzer. In certain instances, the components contained within the "dialysate regeneration modules" can decrease the concentration or conductivity of at least one ionic species, or release and/or absorb at least one solute from a dialysate.

"Dialysis" or "dialysis therapy" is a filtration, or a process of selective diffusion or convection through a membrane.

Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids or by convection through the membrane. The dialysate is used to remove solutes from the fluid to be dialyzed. The dialysate can also provide enrichment to the other fluid.

The term "dialysis mode" can refer to whether the dialysis system or operational settings of the system being used is a single pass dialysis system, a multi-pass system, or combinations thereof.

The term "dialysis system" can refer to a set of components configured to carry out dialysis therapy of any type including peritoneal dialysis, hemodialysis, hemofiltration, hemodiafiltration or ultrafiltration.

The term "dialysate temperature" refers to the temperature of the dialysate at any point during treatment.

"Dialysis treatment duration" can refer to an expected or actual length of time during which dialysis therapy is carried out on a subject.

The term "dialyzer" can refer to a cartridge or container with two flow paths separated by semi-permeable membranes. One flow path is for blood and one flow path is for dialysate. The membranes can be in hollow fibers, flat sheets, or spiral wound or other conventional forms known to those of skill in the art. Membranes can be selected from these materials: polysulfone, polyethersulfone, poly (methyl methacrylate), modified cellulose, or other materials known to those skilled in the art.

"Dialyzer efficiency" can refer to the ability of molecules to be transported through a semi-permeable membrane in a dialyzer.

The term "differential equation" can refer to an equation involving one or more functions and the derivatives of the one or more functions. A derivative is a rate of change of the function at a specific value of a first variable in the equation with respect to a second variable. The second variable is often a time dimension. Differential equations are solved by finding an expression for the function that does not involve derivatives or by use of numerical methods.

The term of "equation" refers to equality containing one or more variables with unknown values. An equation is solved when the values of the variables become known and satisfy the equality. The term of "differential equations" refers to equations involving one or more functions and their derivatives. A derivative is a rate of change of the function at a specific value of a variable in the equation. Differential equations are solved by finding an expression for the function that does not involve derivatives or by use of numerical methods. The term of "functional equation" refers to any equation that specifies a function in implicit form that cannot be reduced to algebraic equations.

The term "flow rate" can refer to the volume or mass of a fluid moving past a specific point per unit of time.

The term "flow rate of bicarbonate" refers to an amount of bicarbonate or bicarbonate predecessors flowed into a dialysate per unit of time.

A "flow through cartridge" can refer to a component through which fluid can be flowed. In one embodiment, the flow through cartridge can contain a dry substance through which a fluid flows, dissolving a portion of the substance to increase the concentration of the substance in the fluid that flows out of the cartridge.

A "fluid" is a gas phase or liquid phase substance optionally having a combination of gas and liquid phases.

The terms "generate" or "generating" refers to producing a result. For example, as used in the context of "generating one or more control algorithms," the terms can refer to the process of using any specified mathematical operations, logical steps, or calculations to result in one or more desired control algorithms whether by automated, semi-automated, hand, or other known methods.

The term "historical value" or "history" can refer to previously calculated or determined quantities. For example, the "historical value" for a patient or group of patients can refer to previously obtained data from a patient, a specified group of patients, or a patient population.

The term "identifying" or to "identify" refers to making a determination of which possible variables meet one or more specific criteria.

The term "infusate solution" or "infusate" can refer to a solution of one or more salts for the adjustment of the composition of a dialysate, such as salts of calcium, magnesium, potassium, and glucose. The term "cation infusate" can refer to an infusate solution containing one or more cations. The infusate solution can also include acids, including citric, acetic, or lactic acid.

An "infusate prescription" is an intended amount of one or more infusate in a dialysate or fluid.

An "infusate profile" can refer to one or more specified infusate concentration, infusate flow rates of a particular infusate, and lengths of time for performing or halting infusate flow. The profile can include any one or more of concentrations of a specified infusate over time and/or volumetric flow. The profile can also refer to a constant or variable rate of infusate flow specific to a particular patient or subject over time and/or volumetric flow. The profile can include plural rates, start/stop instructions. The variable rate described in the profile can include an instantaneous flow rate that is a function of time, such as a curve or a line, or a set period of time. The profile can include a period of time wherein no addition of infusate flow is required.

The terms "input" or "inputting" refer to entering data into a system or into an algorithm by hand, computer, or any known automated or computer implemented method.

The term "iterative algorithm" can refer to calculations repeated a set number of times or until some predetermined result is achieved.

The term "linear interpolation" can refer to a change in rate that varies as a linear function of time.

A "linear transfer function" is a mathematical function used to fit the relationship between inputs and outputs in a linear fashion.

The term "liquid concentrate" can refer to a fluid having a concentration of one or more substances higher than a concentration to be used in a resulting fluid obtained from the liquid concentrate.

A "look up table" is an electronic or non-electronic table correlating the effects of changing a particular variable or variables on an outcome.

The terms "main effect" or "main effects" refer to an effect of an independent variable on a dependent variable, without taking into account the effects of interactions between other independent variables. In general, there is one main effect for each independent variable in a study. The main effect is distinguished from interactions effects. An interaction arises when the effect of the independent variable changes depending on the level of another independent variable.

The term "mathematical model" can refer to a system using mathematical concepts and language. Mathematical models usually contain relationships and variables. A mathematical model can contain one or more differential equations that describe one or more chemical, physical, or physiological processes.

The terms "meter," "metered," "metering in," and the like refer to a measured and controlled addition of a substance, gas, fluid, or combinations thereof.

To "modulate" or "modulates" refers to changing or varying a desired parameter. In one non-limiting example, the phrase "modulates an amount of bicarbonate" in dialysate can refer to changing a bicarbonate concentration in a dialysate by either removing bicarbonate from the dialysate or by adding bicarbonate to the dialysate depending on a chemical state of a component or a history of chemicals flowed through the component.

The term "non-linear interpolation" can refer to a change in rate that varies as a non-linear function of time.

A "non-linear transfer function" is a mathematical function used to fit the relationship between inputs and outputs in a non-linear fashion.

"Numerical methods" are methods of approximating solutions for differential equations and can include the Euler method, the backward Euler method, and the first order exponential integrator method.

The terms "obtain," "obtaining," and the like can refer to receiving information or data from any source by any suitable transmission method such as electronic connection, wireless transmission, automated query and receipt, internet connectivity, database query, hand-entry, API, webhook, or other suitable methods known to those of skill in the art.

An "ordinary differential equation" is a differential equation containing one or more functions of one independent variable and the derivatives of the function.

The term "parameter" of a subject represents a characteristic, feature, or measurable factor that helps define the subject. A parameter can have a nominal description to define the subject qualitatively, and can also be a quantitative value or a certain range of quantitative values, regarding a certain feature of the subject. For example, a parameter of a patient can describe any features related to the patient, including concentration of a certain component in the blood, such as "blood urea nitrogen," "total body water," which can be calculated anthropometrically using the Hume-Weyers equations depending on gender, height, and weight of the individual of interest. A parameter of a hemodialysis system can describe any features of any device or fluid in the system. In statistics, a parameter is often used interchangeably with the terms of "variable" and "factor." A variable denotes a mathematical object, representing value that can be specified and can be varied. Parameters can also be the names of variables in the function.

A "partial differential equation" is a differential equation that contains unknown multivariable functions and their partial derivatives.

A "patient" or "subject" is a member of any animal species, preferably a mammalian species, optionally a human. The subject can be an apparently healthy individual, an individual suffering from a disease, or an individual being treated for a disease.

"Patient acid generation" can refer to the rate at which a patient generates acids due to metabolism of one or more substances in the blood of the patient.

"Patient bicarbonate consumption rate" can refer to the rate at which bicarbonate in the blood of a patient is metabolized.

"Patient parameters" are any parameters or characteristics determined based on measurements taken of a patient.

The term "patient post-dialysis bicarbonate level" can refer to the concentration of bicarbonate ions in a patient after dialysis treatment.

The term "patient pre-dialysis bicarbonate level" can refer to the concentration of bicarbonate ions in a patient prior to a dialysis treatment.

"Patient pre-dialysis urea level" can refer to a measurement of the total urea in the patient prior to dialysis treatment. The patient pre-dialysis urea level can refer to direct measurements of urea, or to measurement of patient blood urea nitrogen, which is a measure of nitrogen in the blood of a patient that comes from urea. The BUN measurement is given in units of mg/dl.

"Patient pre-dialysis weight" can refer to the total weight of a patient prior to dialysis treatment.

"Patient bicarbonate level" can refer to the concentration of bicarbonate ions in a patient.

The term "personalized bicarbonate addition profile" can refer to a constant or variable rate of addition of bicarbonate from a bicarbonate source into a dialysate specific to a particular patient or subject over time and/or volumetric flow. The variable rate can include an instantaneous flow rate that is a function of time, such as a curve or a line, or a set period of time. The profile can also refer to a constant or variable rate of flow specific to a particular patient or subject over time and/or volumetric flow. The profile can include a period of time wherein no addition of bicarbonate is required.

The term "population average" can refer to a quantity for a particular parameter determined on the basis of the general population or a subset of the general population sharing one or more characteristics.

The term "post-dialysis" can refer to an end of a dialysis therapy session. The dialysis therapy session can include plural instances of dialysis therapy delivered throughout a single session and can also include any inter-dialytic periods of time between instances of dialysis delivered during the single session. As such, the term "post-dialysis" refers to the complete end of one dialysis therapy session, which includes all dialysis and inter-dialytic periods that in sum comprise a single dialysis therapy session.

The term "pre-dialysis" can refer to a beginning of a dialysis therapy session wherein the dialysis therapy session includes plural instances of dialysis therapy delivered throughout a single session and also includes any inter-dialytic periods of time between dialysis delivered during the single session. As such, "pre-dialysis" does not refer to the beginning of an instance of dialysis that occurs within the session, such as an inter-dialytic period, but rather to the beginning of the overall dialysis therapy session.

The term "predetermined threshold" can refer to a predetermined value for a parameter, wherein an action taken can depend on the pre-set threshold value of the parameter.

The terms "predicting," and to "predict" refer to the ability to estimate an outcome based on parameters or a set of initial inputs.

A "predictor" is a function that provides an approximation of an output based on estimated values for an input.

The term "processor" as used herein is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. The terms can include ROM ("read-only memory") and/or RAM ("random-access memory") associated therewith.

The term "profile" can refer to a function that varies with time and/or volumetric flow. The profile can include any one or more of plural rates, start/stop instructions, and length of time. The profile can also refer to a constant or variable rates specific to a particular patient or subject over time and/or volumetric flow. The profile can further include functions such as a curve or a line, or a set period of time.

"Setting," "to set," and the like, can refer to an act whether implemented by a processor, computer, or algorithm wherein a parameter or flow rate is controlled to result in a desired result.

The term "significant parameters" refers to initial set of variables or parameters which have a substantially large and statistically significant effect on a particular outcome.

A "sodium prescription" is an intended sodium concentration in the dialysate or blood.

A "sodium profile" is a concentration of sodium in the dialysate as a function of time and/or volumetric flow. The profile can describe sodium concentration during a therapy. The profile can include any function of time, such as a curve or a line, or a set period of time. The profile also can describe a period of time wherein no sodium is present.

The terms "sorbent cartridge" and "sorbent container" refer to a cartridge containing one or more sorbent materials for removing specific solutes from solution, such as urea. The term "sorbent cartridge" does not require the contents in the cartridge be sorbent based, and the contents of the sorbent cartridge can be any contents that can remove waste products from a dialysate. The sorbent cartridge may include any suitable amount of one or more sorbent materials. In certain instances, the term "sorbent cartridge" can refer to a cartridge which includes one or more sorbent materials in addition to one or more other materials capable of removing waste products from dialysate. "Sorbent cartridge" can include configurations where at least some materials in the cartridge do not act by mechanisms of adsorption or absorption.

The term "sorbent cartridge layer order" can refer to the sequential placement of materials within a sorbent cartridge through which dialysate flows.

The term "sorbent cartridge mass" can refer to the mass of one or more materials housed within a sorbent cartridge.

The term "sorbent cartridge pH" can refer to the concentration of hydrogen ions in the sorbent cartridge effluent stream.

The term "sorbent cartridge profile," where profile is used in the context of a sorbent cartridge, refers to any measurable data regarding a sorbent cartridge, including the type or amount of solutes flowed through the sorbent cartridge, identification of patients that have used a sorbent cartridge, a number of times a sorbent cartridge has been used, or any other data regarding a particular sorbent cartridge.

The term "step" or "steps" as used to describe a variable rate of addition, can refer to a profile that includes a first rate of addition for a set period, followed by at least one different rate of addition for a set period. The "steps" can also refer to a rate of addition that increases or decreases over time.

"System parameters" are any parameters or characteristics determined based on a dialysis system. System parameters can include characteristics of any component within the system or variables used in dialysis therapy.

The terms "treating," "treatment," and "therapy" refer to the management and care of a patient having a pathology or condition by administration of one or more therapy contemplated by the present disclosure. Treating also includes administering one or more methods of the present disclosure or using the systems, devices or compositions of the present disclosure in the treatment of a patient. As used, "treatment" or "therapy" can refer to both therapeutic treatment and prophylactic or preventive measures. "Treating" or "treatment" does not require complete alleviation of signs or symptoms, requires no cure, and includes protocols having only a marginal or incomplete effect on a patient.

The term "two-way interactions" describe a relationship between an independent variable and a dependent variable, moderated by a third variable. An independent variable can be any parameters of a patient and of a device, and a dependent variable can be any other parameters having a value depending on the independent variable.

The term "ultrafiltration profile" can refer to one or more specified ultrafiltration rates and lengths of time for performing or halting ultrafiltration to result in a description of the ultrafiltration being performed. The profile can include any one or more of plural rates, start/stop instructions, and length of time to be used during therapy, wherein any can vary as a function of time and/or volumetric flow. The profile can also refer to a constant or variable rates specific to a particular patient or subject over time or volumetric flow. The variable rate can include an instantaneous flow rate that is a function of time, such as a curve or a line, or a set period of time. The profile can also include a period of time wherein no ultrafiltration is required.

"Ultrafiltration rate" can refer to the rate at which fluid is removed from a patient during therapy.

An "update" of a parameter is a change in a value of the parameter from an initial value based on changes in data for the parameter.

"Urease" is an enzyme that catalyzes the conversion of urea into carbon dioxide and ammonium ions.

The term "user specified" can refer to a parameter or variable predetermined by a user and used during dialysis treatment.

*Dialysis device*

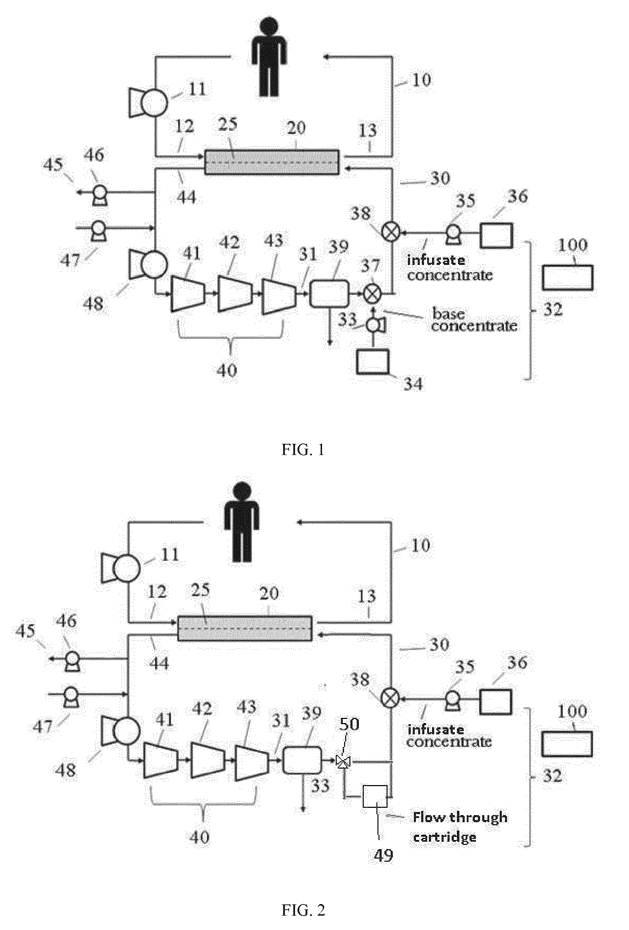

FIG. 1 shows a dialysis system including a dialysis apparatus or system including a blood circuit **10,** a dialysate flow path **30,** and a dialyzer **20** containing a membrane **25.** The blood enters the dialyzer **20** through a blood inlet line **12** and exits through a blood outlet line **13** with a blood pump **11.** The dialysate flow path **30** can be a controlled compliant flow loop. Regenerated dialysate **31** can be in fluid communication with a reconstitution system **32,** which is a source of one or more infusates for addition to the dialysate. The reconstitution system **32** can include a bicarbonate pump **33** and a bicarbonate source **34** containing bicarbonate or bicarbonate predecessors as liquid, dry powder, highly concentrated media, or any combinations thereof. The bicarbonate source **34** can contain concentrated bicarbonate buffer or dry bicarbonate buffer components. The bicarbonate source **34** can also contain other components not limited to the examples described above. The bicarbonate pump **33** can control addition of bicarbonate to the dialysate by metering in bicarbonate or bicarbonate predecessors as a liquid concentrate to the dialysate from the bicarbonate source **34** to achieve a specified dialysate bicarbonate concentration or a specified patient bicarbonate level.

The reconstitution system **32** can also include cation pump **35,** which is fluidly connected to a cation concentrate reservoir **36** containing a cation concentrate in a form as liquid, dry powder, highly concentrated media, or any combination thereof. The cation concentrate reservoir **36** can contain calcium, potassium, and magnesium cation concentrates. The cation concentrate reservoir **36** can also contain acetate concentrates and serve as an acetate concentrate source. For example, the cation concentrate reservoir **36** can contain a mixture of calcium chloride, calcium acetate, potassium chloride, potassium acetate, magnesium chloride, and magnesium acetate, or acetic acid. The cation concentrate can include alternative anions, such as citrate or lactate anions. The cation concentrate reservoir **36** can also contain glucose. Additionally, the cation concentrate reservoir **36** can contain sources of acids, such as citric, acetic, or lactic acid. The purpose of the reconstitution system **32** is to rebalance and provide the appropriate electrolytes and buffer to the dialysate. The cation and acetate concentrates can be contained in separate containers, such as separate acetate concentrate sources and cation concentrate sources, and need not all be in the single cation concentrate reservoir **36.** For example, two, three, four, or more reservoirs containing concentrates can be provided.

The bicarbonate source **34** can include a container containing a concentrated buffer solution, such as sodium bicarbonate buffer solution. The bicarbonate source **34** can alternatively contain sodium carbonate, or mixtures of sodium bicarbonate and sodium carbonate. The bicarbonate source **34** can also include potassium carbonate, potassium bicarbonate, or mixtures thereof in the same or different container as the sodium bicarbonate. The system may also include an additional container containing other solutions, such as a solution of calcium acetate, magnesium acetate and potassium acetate.

The reconstitution system **32** can further include a dry sodium bicarbonate cartridge containing sodium bicarbonate (not shown), where a sodium bicarbonate concentrate can be produced by metering a portion of the regenerated dialysate **31** through the dry sodium bicarbonate cartridge, and then be re-infused to the dialysate to add bicarbonate as needed.

The reconstitution system **32** can support using a pre-mixed liquid sodium bicarbonate concentrate and using the dry powder cartridge. Further, one skilled in the art would understand that more than one reconstitution system **32** may be included in the hemodialysis apparatus.

Optionally, the bicarbonate or bicarbonate predecessors can be pumped into, and subsequently mixed with, the regenerated dialysate **31** through a mixer **37,** and the cation concentrate can be pumped into, and subsequently mixed with the regenerated dialysate **31** through a mixer **38.** However, additional optional mixers may be provided for mixing the bicarbonate or the cation concentrate with the regenerated dialysate **31.** The mixers **37** and **38** may also be replaced with one mixer to mix the cation concentrate and the bicarbonate with the regenerated dialysate **31.** The mixers **37** and **38** can be static mixers, which are separate components capable of mixing fluids, or may instead be the infusion point in the dialysate flow path **30** where the bicarbonate and cation concentrate are infused. The infusion of the bicarbonate and cation concentrate may cause mixing with the dialysate to ensure complete mixing.

Gas can be removed from the regenerated dialysate **31** when passing through at least one degasser **39.** The at least one degasser **39** may be positioned to operate prior to the addition of the base concentrate, but also may be positioned elsewhere in the dialysate flow path **30.**

FIG. 1 also shows a dialysate regeneration module **40** containing sorbent materials to regenerate the dialysate. The dialysate regeneration module **40** can be a sorbent cartridge. In the dialysis system according to the invention as claimed, the dialysate flow path includes a sorbent cartridge receiving spent dialysate from the dialysate flow path for regeneration and return to the dialysate flow path.

The dialysate regeneration module **40** can contain multiple materials selected from the group: 1) a urease-containing material **41,** where urease catalyzes the conversion of urea to ammonium ions and carbon dioxide; 2) a zirconium phosphate (ZrP) material **42** with the capacity to act as a cation exchanger by absorbing a large quantity of ammonium ions, potassium ions, calcium ions and magnesium ions for sodium and hydrogen ions; 3) a hydrous zirconium oxide material (ZrO) **43,** which acts as an anion exchanger, such as by exchanging phosphate for acetate; 4) an activated carbon material (not shown) with a surface area for adsorption of a wide range of impurities, including metal ions and uremic toxins, such as uric acid, creatinine, and $\beta$2-microglobin; and 5) other ion-exchange materials (not shown) for removing cations and anions Ion-exchange materials can include weak and strong acid cation exchange resins, weak and strong basic anion exchange resins, chelating ion exchange resins, or other ion exchange resins known to those skilled in the art. The materials present in the dialysate regeneration module **40** may be arranged in layers in any order, or can be intermixed. Alternative methods of dialysate regeneration can be used, including an electrodialysis unit, or modules that do not include enzymatic conversion of urea to bicarbonate. The dialysate regeneration module **40** can modulate the amount of bicarbonate in the dialysate, either by removing bicarbonate, converting bicarbonate to other substances, or by adding bicarbonate to the dialysate depending on the starting state and the history of chemicals pumped through the dialysate regeneration module **40.** The history can be obtained from sensor data and electronic database(s) (not shown) receiving and recording the operation and performance of the dialysate regeneration module **40,** or other suitable methods known to those of skill in the art. The algorithms and control described can also work with other dialysis modes, including single-pass dialysis systems or combinations of single and multi-pass systems.

The regenerated dialysate emerging from line **31** in FIG. 1 can pass through the dialyzer **20** and exit as spent dialysate **44.** The spent dialysate **44** can pass an ultrafiltration system **45** including an ultrafiltration pump **46** and an ultrafiltration reservoir (not shown). The ultrafiltration system **45** can remove ultrafiltrate obtained across the dialyzer from the patient and remove any other fluid added into the dialysate flow path **30,** such as fluid from the reconstitution system **32.**

At least a portion of the spent dialysate **44** can flow through the dialysate regeneration module **40** with a dialysate pump **48.** The spent dialysate **44** can be supplied with source water through a water pump **47** prior to reaching the dialysate regeneration module **40.** Source water can come from tap water or other types of water resources.

The apparatus in FIG. 1 can further include or communicate with a controller **100,** which controls the bicarbonate pump **33** to introduce the bicarbonate concentrate into the regenerated dialysate **31.** In any embodiment the controller **100** can be a processor in communication with the bicarbonate pump **33.** The controller **100** can also control the cation pump **35** for adding the cation concentrate into the regenerated dialysate **31.** One skilled in the art would understand that the controller **100** can further control other components and operations of the hemodialysis apparatus. The controller **100** can further manage patient acid-base status by controlling the addition of the base concentrate and the cation concentrate to the dialysate. The controller can include one or more processors, memory, and input/output interfaces. The memory can be in communication with the processor and store instructions that when executed perform the methods of the present disclosure. The input/output interface can include an input port to receive the patient and system parameters, information from one or more sensors including conductivity sensors, and any other information that can be entered by a patient or health care professional. The input/output interface can also have an output interface to output a calculated bicarbonate addition rate or profile. Intervening components, such as the input/output interface can be present between the processor and the bicarbonate source **34.**

FIG. 2 illustrates an alternative dialysis system to FIG. 1. Similar components in each figure are represented by the same reference numbers. In FIG. 2, the system uses a bicarbonate flow through cartridge **49** in place of the liquid concentrate infusion system illustrated in FIG. 1. The flow through cartridge **49** includes dry bicarbonate. As dialysate is pumped through the flow through cartridge, a portion of the dry bicarbonate is dissolved, increasing the bicarbonate concentration. Valve **50,** or any other type of regulator, controls the amount of dialysate that passes through the flow through cartridge **49,** controlling addition of bicarbonate added to the dialysate flow path **30.** The algorithms described can be implemented with the system of FIG. 1 that uses a liquid concentrate to control the bicarbonate addition, and the system illustrated in FIG. 2, which uses a flow through cartridge.

*Bicarbonate control*

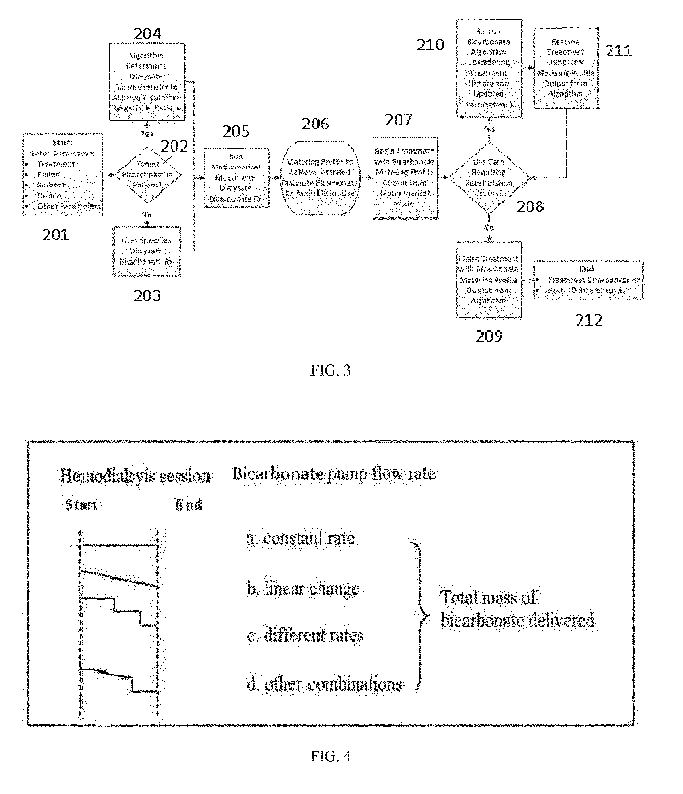

FIG. 3 illustrates a bicarbonate control algorithm for controlling bicarbonate addition to a dialysate flow path. In step **201,** one or more patient parameters and one or more parameters affect the dialysate bicarbonate concentration as well as the patient bicarbonate concentration. Table 1 illustrates several dialysis and patient parameters that can be entered into the system. As illustrated in Table 1, the dialysis parameters used by the system can include any one or more of dialysis treatment duration, ultrafiltration rate and/or profile, dialysate flow rate and/or profile, blood flow rate and/or profile, sodium prescription and/or profile, infusate prescription and/or profile, sorbent cartridge mass, sorbent cartridge layer order, sorbent cartridge pH and/or profile, dialyzer efficiency, degasser outlet PCO$_2$ levels and/or profile, whether bicarbonate is to be metered by a flow-through cartridge or liquid concentrate, dialysis mode, and dialysate temperature. Because the amount of CO$_2$ removed by the degasser may be variable, the algorithms can use a PCO$_2$ profile and are not limited to a fixed PCO$_2$ value. Sample units are provided in Table 1, however, one of skill in the art will understand that the units provided are exemplary and can be freely converted to any other units, so long as the numerators and denominators in the equations represent proper dimensions. The patient parameters used by the system can include any one or more of patient pre-dialysis bicarbonate level, patient pre-dialysis urea level, patient pre-dialysis weight, patient acid generation, and patient bicarbonate consumption rate. The bicarbonate levels can include serum bicarbonate levels. The bicarbonate levels can be measured by any means known in the art, including direct measurement by a blood draw, a measure of serum CO$_2$, which is correlated to serum bicarbonate, photometric tests, enzymatic tests, or any other method that provides a measurement of the bicarbonate level in a patient. The bicarbonate measurement can also be based on a measurement of patient lactate levels, which is negatively correlated with patient bicarbonate level. The pre-dialysis urea level can be either a direct measurement of urea in the patient, or can be a measurement of blood urea nitrogen (BUN).

Table 1

| Input Label |
| --- |
| Hemodialysis treatment duration (minutes) |
| Hemodialysis treatment ultrafiltration rate(s) and/or profile(s) (mL/min) |
| Hemodialysis treatment dialysate flow rate(s) and/or profile(s) (mL/min) |
| Hemodialysis treatment blood flow rate(s) and/or profile(s) (mL/min) |
| Hemodialysis treatment sodium prescription(s) and/or profile(s) (mmol/L) |
| Hemodialysis treatment infusate prescription(s) and/or profile(s) (mmol/L) |
| Patient pre-dialysis bicarbonate level(s) (mmol/L) |
| Patient pre-dialysis urea level(s) (mmol/L) |
| Patient pre-dialysis weight(s) and/or total body water volume(s) (L) |
| Patient acid generation and/or bicarbonate consumption rate(s) (mol/min) |

(continued)

| Input Label |
|---|
| Sorbent cartridge mass(es) and/or layer order(s) (kg) |
| Sorbent cartridge pH level(s) and/or profile(s) (pH) |
| Device dialyzer efficiency (K0A) (L/min) |
| Device degasser outlet PCO2 level(s) and/or profile (mmHg) |
| Whether bicarbonate is to be metered by a flow-through cartridge or liquid concentrate (Y/N) |
| Dialysis mode (single pass or multi-pass) |
| Dialysate temperature (°C) |

Several variables presented in Table 1 include profiles. The quantitative variables need not be constant, and can be changed by the system throughout therapy. A profile includes the changes to a variable over time and/or volumetric flow during dialysis treatment. As an example, an ultrafiltration profile may include a first ultrafiltration initially during therapy, and a higher or lower rate during a later point in time of the therapy. Any combination of ultrafiltration rates, periods of time, and increases or decreases of such rates can be used. The system can also account for changes to the ultrafiltration rate or other parameters in calculating the rate and length of time period of bicarbonate addition or withholding. That is, the system can use any combination of a first rate, that can subsequently accelerate or decrease, and then halt and restart to result in a desired ultrafiltration profile. Similarly, the system can use a dialysate flow profile, with the dialysate flow rate varying during therapy. The system can account for changes to the dialysate flow rate throughout the therapy.

The system can operate to achieve a target patient bicarbonate concentration or a target dialysate bicarbonate concentration. In step **202,** the user can instruct the system of which target to use. If the user specifies a dialysate bicarbonate concentration as the target, the target dialysate bicarbonate concentration can be entered in step **203.** If the user specifies a target patient bicarbonate concentration, the target patient bicarbonate concentration can be entered in step **204,** and the system can utilize an algorithm, such as a differential equations based mathematical model, to set the dialysate bicarbonate prescription to result in the target patient bicarbonate concentration. The system can use an iterative algorithm with an initial guess of the dialysate bicarbonate prescription or profile to set the dialysate bicarbonate prescription or profile based on the user specified patient bicarbonate level. The system can calculate the bicarbonate prescription or profile and set a refined dialysate bicarbonate concentration. The system can then use the calculated dialysate bicarbonate concentration to calculate the patient bicarbonate level that will result. The calculations can be repeated until the algorithm undergoes an iterative convergence or until the calculated patient bicarbonate level and dialysate bicarbonate level are within a predetermined threshold. Alternatively, the system can use a paper or electronic look up table, using the patient and system parameters as well as the target bicarbonate levels to obtain a bicarbonate flow rate.

After obtaining a dialysate bicarbonate prescription in either step **203** or **204,** the system runs a mathematical model with the dialysate bicarbonate prescription using the dialysis and patient parameters in step **205** to set a personalized bicarbonate addition profile in step **206.** The personalized bicarbonate addition profile is the amount, rate, and timing of bicarbonate addition from the bicarbonate source into the dialysate necessary to achieve the dialysate bicarbonate prescription. Dialysis treatment begins in step **207** using the bicarbonate addition profile obtained in step **206.** The personalized bicarbonate addition profile can include a constant bicarbonate addition rate, such as a specified rate to be used for the bicarbonate addition throughout therapy, or can include a bicarbonate addition profile having a variable bicarbonate addition rate. The personalized bicarbonate addition profile having a variable bicarbonate addition rate can include steps, linear interpolations, non-linear interpolations or a continuous addition of bicarbonate from the bicarbonate source into the dialysate.

In any embodiment, the system can determine whether a recalculation of the bicarbonate metering profile is necessary during dialysis treatment in step **208.** A recalculation may be necessary if any of the dialysis and/or patient parameters change unexpectedly during treatment. The recalculation can be based on an update of any parameters and/or use conditions during treatment, including but not limited to determination of online clearance, dialysate pH, conductivity of the dialysate, and/or changing of blood flow rates. As an example, if online clearance is determined to be low, a corresponding change in the bicarbonate calculation may be necessary based on the updated online clearance. A conductivity sensor positioned in the dialysate flow path can determine the conductivity of the dialysate and transmit the information to the processor or control system. For online clearance, the algorithm can use a measured value of clearance obtained during treatment to update the calculated value provided by Eq(13). The algorithm can be re-run using the history of the bicarbonate metering profile used up to an integration time-point of the clearance measurement using the measured rather than calculated clearance. The measured clearance can then be used for the remainder of the integration time with the same bicarbonate dialysate and/or patient targeting scheme originally chosen. As an additional example, if the prescribed blood flow rate is changed from 300 mL/min to 250 mL/min one hour into dialysis treatment, the algorithm can recalculate the bicarbonate metering profile based upon the history of 300 mL/min for one hour, the current value of 250 mL/min, and the anticipated future value of 250 mL/min. The system can use the updated schedule to refine the dialysate bicarbonate accuracy.

If recalculation is unnecessary or not desired, the system can finish the dialysis treatment in step **209** using the bicarbonate metering profile. If recalculation is necessary, the system can recalculate the bicarbonate metering profile in step **210** considering the treatment history and updated parameters. Treatment is resumed in step **211** using the new bicarbonate metering profile output from the algorithm in step **210.** The system can again determine whether recalculation is necessary in step **208.** After treatment, the process can end in step **212** with the desired bicarbonate prescription or patient post-dialysis bicarbonate concentration.

FIG. 4 illustrates different methods (not claimed) for delivering bicarbonate concentrate into the dialysate over the course of treatment. Curve (a) of FIG. 4 shows that a bicarbonate concentrate can be introduced to the dialysate with a constant addition throughout the therapy session. Curves (b)-(d) of FIG. 4 show that the flow rate of bicarbonate can include a time-varying profile, which yields an equal amount of total mass of bicarbonate delivered. In non-limiting examples, curve (b) illustrates that the bicarbonate concentrate flow rate can be changed in a linear interpolation, initially delivered at a high flow rate, and decreased linearly with time throughout therapy. Alternatively, the bicarbonate concentrate can initially be delivered at a low flow rate and increase linearly throughout therapy. Curve (c) shows that the bicarbonate flow rate may be changed in steps, such as by starting at a highest end of the desired range at starting of the dialysis session, maintaining the initial bicarbonate concentrate flow rate for a portion of the session, and decreasing the bicarbonate flow rate at discrete times during therapy. Curve (d) shows that the bicarbonate concentrate can also be delivered at a non-linear interpolation, changing the rate to reach the target total mass of bicarbonate in a non-linear fashion. The base concentrate may also be delivered in any combination of a constant rate, a linear interpolation, a non-linear interpolation, or steps to reach the target total mass of bicarbonate delivered. A person skilled in the art will understand that the base concentrate is not limited to the above described methods of delivery.

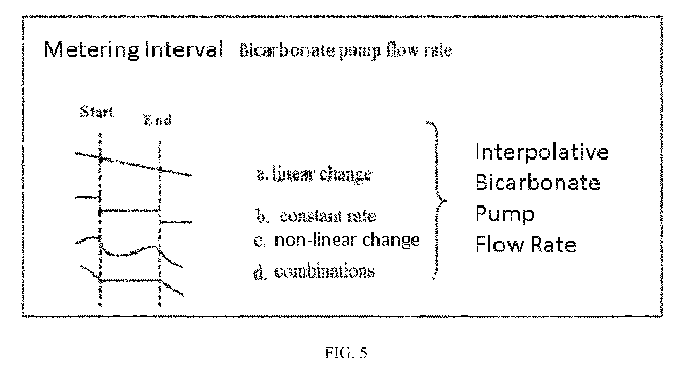

FIG. 5 illustrates interpolations within a specific metering interval, designated as the dashed lines in FIG. 5. The system or algorithm may recommend specific bicarbonate metering flow rates associated with specified time periods during therapy, with the time periods represented by the dashed lines in FIG. 5. The system can modify the bicarbonate flow rate in any fashion between the two specific time periods. For example, the system can fit a single average value to the metering profile to obtain a constant metering rate throughout the therapy, illustrated as curve (b) in FIG. 5. The system can alternatively fit a linear equation to the metering profile to obtain a linear rate, illustrated as curve (a). The system can fit non-linear functions, such as polynomials, splines or other non-linear functions to obtain a non-linear metering profile, illustrated as curve (c). The system can also fit a series of step functions to obtain a stepped rate (not shown in FIG. 5), or any combination of functions between the metering interval set points.

In systems using a lookup table to obtain the personalized bicarbonate flow rate, the lookup table can provide specific bicarbonate flow rates at specific points in time [t, Qadd(t)]. Between the specific time points obtained from the lookup table, the system can vary the bicarbonate flow rate in any manner described, including linear interpolations, non-linear interpolations, constant rates, steps, or any combination thereof. For example, the table may provide a specific bicarbonate flow rate at time = 5 minutes and at time = 15 minutes. In between provided values, the system can use a number of interpolation schemes as depicted in FIG. 5, using the known values in the table.

*Bicarbonate Control Calculations*

The mass of any arbitrary species in the dialysate infused immediately before the dialyzer at the inlet of the dialyzer can be given by Eq(1).

$$M_d = M_{eff} + M_{add} \qquad\qquad Eq(1)$$

$M_d$ is the mass of the species in the dialysate at the inlet of the dialyzer, $M_{eff}$ is the mass of the species in the sorbent cartridge effluent and $M_{add}$ is the mass of the species added to the dialysate. In a non-limiting example of mass infusion by liquid concentrate, the mass of the species in the effluent can be given by Eq(2), the mass of the species in the dialysate is given by Eq(3), and the mass of the species added is given by Eq(4):

$$M_{eff} = C_{eff} * (Q_d - Q_{add}) \qquad\qquad Eq(2)$$

$$M_d = C_d * Q_d \qquad\qquad Eq\ (3)$$

$$M_{add} = C_{add} * Q_{add} \qquad\qquad Eq\ (4)$$

$C_{eff}$ is the concentration of the species in the effluent, $Q_d$ is the flow rate of the dialysate, $Q_{add}$ is the flow rate of the infusion of the species into the dialysate, $C_d$ is the concentration in the dialysate, $C_{add}$ is the concentration of the species in the fluid added to the dialysate. As used herein, the variable Q refers to the volumetric flow rate of the species noted. One of skill in the art will understand that a system with a flow through cartridge as illustrated in FIG. 2 instead of the infusion system of FIG. 1 can have slightly different equations because no additional volume is being added by infusion and that the mass balance provided in Eq(1) remains valid. Rearranging the equations provides Eq(5), which gives the flow rate of the infusion based on each of the other factors.

$$Q_{add} = Q_d \frac{C_d - C_{eff}}{C_{add} - C_{eff}} \qquad\qquad Eq(5)$$

In single pass dialysis, the spent dialysate is discarded. $C_{eff}$ becomes 0. The concentration of a species in the dialysate is easily controlled by metering a concentrate fluid at concentration $C_{Add}$ and flow rate $Q_{Add}$ into a stream of species-free fluid flowing at $(Q_{Bath} - Q_{Add})$ using a split fraction calculation. In contrast, sorbent dialysis uses adsorption and ion-exchange mechanisms to remove uremic toxins and electrolytes from the dialysate. The dialysate after passing through the sorbent cartridge can have a non-zero concentration of several species, including bicarbonate. The non-zero bicarbonate concentration changes the split fraction calculation that applies to the metering of bicarbonate required to create fresh dialysate at the intended prescription, and requires knowledge of the cartridge effluent bicarbonate concentration at each time point to determine the bicarbonate metering rate $Q_{add}(t)$.

One of skill in the art will understand that additional or alternative variables can influence the dialysate and/or patient bicarbonate level, and can be included in the calculations. Further, many of the patient and dialysis parameters in Table 1 can provide a bicarbonate flow rate.

The description of the physiologic compartmentalization behavior of an arbitrary chemical species is a critical component of a sorbent hemodialysis mathematical model. At the simplest level, a non-limiting example of compartmentalization in the patient can be described by a single well-mixed total body water volume. An example mathematical description of patient volume $V_p$ [L] is provided in Eq(6).

$$\frac{dV_P}{dt} = I_w(1 - Ind) - Q_{UF}(Ind) \qquad\qquad Eq(6)$$

$Q_{UF}$ is the ultrafiltration rate in the dialyzer [L/min], $I_w$ is the water intake rate [L/min] and $Ind$ is a binary indicator variable for dialysis therapy with $Ind = 0$ if dialysis is not occurring, and $Ind = 1$ if dialysis is occurring. With a description of physiologic compartmentalization, a description of solute generation/consumption and transport is needed for arbitrary chemical species. An example mathematical description for arbitrary dissolved chemical species "$i$" in the patient by a dynamic mass balance is provided in Eq(7).

$$\frac{dM_{P,i}}{dt} = G_{P,i} - (Ind)J_i(V_P, M_{P,i}, C_{Di,i}) + R_{P,i} \qquad\qquad Eq(7)$$

$M_{p,i}$ is the mass of the species "$i$" in the patient [mole], $G_{p,i}$ is the generation rate of the of the species "$i$" in the patient [mole/min], $J_i$ is the total mass transfer rate of species "$i$" from the patient into the dialysate [mole/min], $C_{Di,i}$ is the concentration of species "$i$" in the regenerated dialysate when the regenerated dialysate enters the dialyzer [M], and $R_{P,i}$ is the production rate of species "$i$" due to chemical reactions [mole/min]. The concentration of species "$i$" in the patient ($C_{p,i}$ [M]) is assumed to equal the concentration of species "$i$" flowing into the dialyzer ($C_{Bi,i}$ [M]), which can be calculated via Eq(8).

$$C_{Bi,i} = \frac{M_{P,i}}{V_P} \qquad\qquad Eq(8)$$

An example of the transport of an arbitrary solute "*i*" through a dialysis membrane by diffusion and convection is provided in Eq (9).

$$J_i = D_i \left( C_{Bi,i} - C_{Di,i} \right) + Q_{UF} C_{Bo,i}$$  Eq(9)

$D_i$ is the diffusive dialysance of species "*i*" [L/min] and $C_{Bo,i}$ is the concentration of species "*i*" in the blood flowing out of the dialyzer [M]. Applying Equation (4) requires the knowledge or calculation of the blood outlet concentration $(C_{Bo,i})$. However, since the ultrafiltration rate is usually small compared to the blood flow rate $(Q_{UF} << Q_B)$, a simplifying approximation can be made to obtain Eq(10), which conveniently does not include $C_{Bo,I}$.

$$J_i = D_i \left( C_{Bi,i} - C_{Di,i} \right) + Q_{UF} \left[ C_{Bi,i} - \frac{D_i}{Q_{Bi}} \left( C_{Bi,i} - C_{Di,i} \right) \right]$$  Eq(10)

The diffusive dialysance $(D_i)$ can be calculated as a function of blood and dialysate flow rates and the mass transfer coefficient for species "*i*" $(K_oA_i, mL/min)$.

$$D_i = Q_B \frac{e^{K_0A_i\left(\frac{Q_D-Q_B}{Q_DQ_B}\right)} - 1}{\left( e^{K_0A_i\left(\frac{Q_D-Q_B}{Q_DQ_B}\right)} - \frac{Q_B}{Q_D} \right)}$$  Eq(11)

A non-limiting example of a dynamic mass balance for the fluid inside the dialysate circuit $(V_{dialysate}, [L])$ assuming that the bicarbonate is metered by a liquid concentrate is provided in Eq(12).

$$\frac{dV_{dialysate}}{dt} = 0 = Q_{UF} + Q_{tap} + Q_{base} + Q_{acid} - Q_{waste}$$  Eq(12)

$Q_{tap}$ is the volumetric flow rate of source water intake from outside of the dialysate flow path by water pump 47 as illustrated in FIG. 1 which can be set to a value that results in the intended prescription of sodium in the dialysate flow path 30. $Q_{base}$ (also known as $Q_{add}$) is the flow rate of base solution from bicarbonate source 34, and can be constrained to satisfy Eq(5) for the intended prescription of bicarbonate in the dialysate flow path 30. $Q_{acid}$ is the flow rate of fluid from cation concentrate reservoir 36. $Q_{waste}$ is the flow rate of fluid out of the dialysate flow path by ultrafiltration pump 46. Eq(13) can be derived from Eq(12).

$$Q_{waste}(t) = Q_{UF} + Q_{tap}(t) + Q_{acid} + Q_{base}(t)$$  Eq(13)

Using the system of FIG. 1, Eq's(14) and (15) can be derived from Eq(13).

$$Q_{mix} = Q_D - Q_{acid}$$  Eq(14)

$$Q_{col}(t) = Q_{mix} - Q_{base}(t)$$  Eq(15)

$Q_{mix}$ is the flow rate of fluid in the dialysate flow path between mixers 37 and 38, while Qcol is the flow rate of fluid through the dialysate regeneration module 40. Qd is the flow rate of fluid at the inlet of the dialyzer. Examples of dynamic mass balances for the arbitrary solute "*i*" in the three control volumes are provided in Equations (16), (17), and (18). The subscripts *D, left,* and *right* refer to the dialyzer 20, left-hand side of tubing from the dialyzer 20 to the start of the dialysate regeneration module 40, and right-hand side of the tubing from the mixer 38 to the dialyzer 20, respectively. The dialysate regeneration module 40, degasser 39, and base concentrate mixer 37 can be modeled as pseudo steady-state functions and do not explicitly require volumes of dialysate in the example model. The physical volumes of the three elements can be lumped into $V_{left}$ and/or $V_{right}$.

$$\frac{dM_{D,i}}{dt} = V_D \frac{d\overline{C}_{D,i}}{dt} \approx \frac{V_D}{2}\left(\frac{dC_{Di,i}}{dt} + \frac{dC_{Do,i}}{dt}\right) = J_i + Q_D C_{Di,i} - \left(Q_D + Q_{UF}\right)C_{Do,i} + R_{D,i} \qquad \text{Eq(16)}$$

$$\frac{dM_{left,i}}{dt} = V_{left}\frac{dC_{colin,i}}{dt} = \left(Q_D + Q_{UF}\right)C_{Do,i} + Q_{tap}C_{tap,i} - Q_{waste}C_{Do,i} - Q_{col}C_{colin,i} + R_{left,i} \qquad \text{Eq(17)}$$

$$\frac{dM_{right,i}}{dt} = V_{right}\frac{dC_{Di,i}}{dt} = Q_{mix}C_{colout,i} + Q_{acid}C_{acid,i} - Q_D C_{Di,i} + R_{right,i} \qquad \text{Eq(18)}$$

Combining Equations (16) and (18) provides the concentration of solute "*i*" at the dialyzer outlet in Eq(19).

$$\frac{dC_{Do,i}}{dt} = \left(\frac{2}{V_D}\right)\left[J_i + Q_D C_{Di,i} - \left(Q_D + Q_{UF}\right)C_{Do,i} + R_{D,i}\right] - \left(\frac{1}{V_{right}}\right)\left[Q_{mix}C_{colout,i} + Q_{acid}C_{acid,i} - Q_D C_{Di,i} + R_{right,i}\right]\text{Eq(19)}$$

The details of calculating $C_{colout}$, or the concentration at the outlet of the sorbent cartridge, are greatly influenced by the chemical species included in an implementation of the described model, however, by way of non-limiting example, one skilled in the art could use a pseudo-steady state approach considering the concentration of each species, acid-base chemistry, and the titration behavior of the sorbent cartridge layers as a basis for the calculation to give Eq(20).

$$C_{colout} = f\left(C_{colin}, pH_{ZO}, P_{CO_2,degas}, Q_{col}, Q_{Base}, C_{Base,Na}, C_{Base,CO_2,total}\right) \qquad \text{Eq(20)}$$

To calculate the effects of each variable on the bicarbonate concentration in the dialysate and/or the patient bicarbonate level, the system can use any method known in the art, including lookup tables, partial differential equations, ordinary differential equations, and/or any curve fitting method. The curve fitting can include linear and/or non-linear transfer functions, lookup tables, fitting data from design of experiments, and regression analysis including but not limited to linear regression, Bayesian linear regression, neural network regression, decision forest regression, logistic regression, and boosted decision tree regression. Any curve-fitting approaches necessarily approximate the solutions to the differential equations described. Ordinary differential equations can be solved by finding an expression for the function that does not involve derivatives or by use of any numerical methods known in the art. The equations provided herein are ordinary differential equations, which can be extended to partial differential equations by introducing spatial dependence and/or plug-flow terms. As an example pertaining to a lookup table, the example mathematical model and/or a modification of the mathematical model can be run assuming various combinations of the required parameters (e.g. values of 15, 20, 25, 30 mM for pre-dialysis bicarbonate and permutations with 0, 10, 20, 30, 40 mM for pre-dialysis urea and permutations with 300, 400, 500 mL/min for dialysate flow rates), and the bicarbonate metering schedules can be aggregated into a lookup table. One skilled in the art can use any number of interpolation and/or kriging (Gaussian process regression) techniques to generate bicarbonate metering schedules not lying exactly on the grid points. As an example of curve fitting, the mathematical model and/or a modification of the mathematical model described herein could be used to generate a dataset under a number of different parameter assumptions such as different combinations of the pre-dialysis urea level, pre-dialysis bicarbonate level, and other parameters for application of design of experiments methodology, and response-surface methodology, regression based or otherwise, can be used considering the mean bicarbonate metering rate for that set of assumed parameters to apply curve fitting methodology. As an example, a fit curve Y = F(X1, X2, ... XN) could calculate a bicarbonate metering rate (i.e. Y) for a given treatment based upon inputs (i.e. X1, X2, ... XN) including, but not limited to, pre-dialysis bicarbonate, pre-dialysis urea, and/or dialysate flow rate.

The processor or system can also obtain historical values and population averages, and assume one or more parameters based on the historical values and population averages. Population averages (e.g. the average pre-dialysis bicarbonate of patients receiving dialysis is around 22 mM - a fixed value) can be used universally in place of personalized values measured at or immediately before dialysis treatment (e.g. by a blood draw). Historical values (e.g. the average of a given patient's pre-dialysis bicarbonate level over the last 10 treatments) can also be used in place of measured pre-dialysis values to avoid further blood draws. However, the algorithm may be able to provide better control if point-of-care values are used instead of population averages and/or historical values.

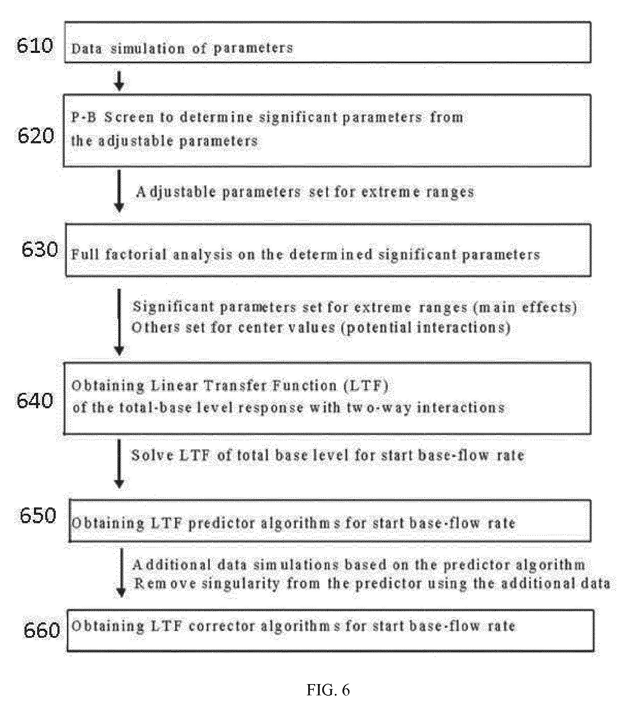

FIG. 6 illustrates a non-limiting example of the steps for generating the open-loop control algorithms in a curve fitting embodiment. One of the steps can be automated, semi-automated, or performed by hand. The steps can be formed into an ensemble of various functions and tools to generate the open-loop control algorithms by curve fitting.

Such ensemble can be a special purpose or specially-adapted computer, software, or processor. At step **610,** simulation data can be collected for a set of adjustable and non-adjustable patient and system parameters by applying a mathematical model. The mathematical model can contain differential equations corresponding to the adjustable and non-adjustable parameters. Adjustable parameters are parameters that have values adjustable within a reasonable range technically or physiologically, such as the infusate prescription or ultrafiltration rate and/or profile to be used in a dialysis session. Non-adjustable parameters are parameters that cannot be changed either technically or physiologically, such as the sorbent cartridge layers. Simulations can be run by the mathematic model under appropriate computational environment. For example, one or more of the simulations can be run on a high-performance computing (HPC) cluster, such as those maintained by Medtronic Design Automation using MATLAB version R2014a (8.3.0.532) for Linux, MATLAB Parallel Computing Toolbox version 6.4, and MATLAB Statistics Toolbox version 9.0. MATLAB is known to be a numerical computing environment and programming language. A job-scheduler can be used for submitting all jobs, for example, the Univa Grid Engine (UGE) job-scheduler. A single node of the HPC cluster can be used as the computing environment. The single node of the HPC cluster may contain two, eight-core 3.3GHz Intel Xeon E5-2667 v2 CPUs (16 cores total) running Red Hat Enterprise Linux 6.4 with Linux kernel 2.6.32-358.el6.x86_64. Simulation output data can be copied over network from the HPC cluster to a local machine, such as a Dell Precision WorkStation T3500; 3.2GHz Intel Xeon W3565 CPU running Windows 7 Enterprise (64-bit version, Service Pack 1). Analyses of data can be performed by any suitable analytical tools, such as MATLAB version R2014b (8.4.0.150421) for Windows with MATLAB Statistics Toolbox version 9.1. The HPC cluster, the single node of the HPC cluster, and the analytical tools that are used for processing the simulation data are not limited to the examples listed above.

In step **620,** a Plackett-Burman (P-B) design of experiments (DOE), also referred to as a P-B screen or P-B design, may be used to identify significant parameters in terms of the effect of these parameters on predicting the post-dialysis patient bicarbonate level. P-B designs, as known in the art, are often used to identify the most important or significant factors among a number of factors of interest about the system, assuming all interactions are negligible when compared with the few important main effects. The significant parameters can also be selected by using other designs of experiments.

After simulated data of adjustable parameters are obtained at step **610,** the P-B design with a response (output) of patient post-HD total base level can be used to screen for the most dominant or significant factors over ranges of the adjustable parameters at step **620.** The ranges of the adjustable parameters can each be set according to corresponding clinical data and technical requirements. A process of determining the parameter ranges can be carried out separately by a user, or can be incorporated into the computer-based process according to pre-stored technical and clinical data. All or a portion of the parameters can undergo the P-B design.

Aliasing between main effects and two-way interactions can be eliminated through proper designs. As known in the art, when the estimate of a main effect of a parameter is influenced by one or more other effects, such as a higher order interaction, the main effect is said to be aliased. To remove the alias, for example, a 2048 run "Resolution IV" P-B design can be formed by "fold over" of a 1024 run "Resolution III" P-B design. A "resolution III design" is known to be a design where main factors are confounded with two factor (i.e. two-way) and higher order interactions, and a "resolution IV design" is a design where main effects are confounded with three factor and higher order interactions and all two factor interactions are confounded with two factor interactions and higher order interactions. The term "fold over" refers to an experimental design technique and is used to provide more data on potential combination of factors by running additional experiments by reversing the signs of the value assigned to all factors, i.e. factors that were at the lower level in the original tests are at high level in the new tests, and vice versa.

In certain embodiments, separate P-B screens can be run depending on the acetate tolerance of the patient, where one P-B screen is for receiving acetate-containing dialysate and the other P-B screen is for receiving acetate-free dialysate. A transfer function of main effects can be fit to the results of each P-B screen. Each transfer function can be used to measure the relative importance of each parameter over an extreme range for the parameter by multiplying the extreme range to the parameter's corresponding transfer function coefficient. As a result of the P-B screens, a portion of the parameters can be identified as significant parameters for predicting the post-dialysis patient bicarbonate level.

As known in the art, the coefficient of determination ($R^2$) is a statistical measure of how close the data are fit to the model. $R^2$ is normally between 0-1, and the higher the $R^2$ value is, the better the linear model fits the data. In an example where 40 parameters including those in Table 1 were screened, $R^2$ was equal to 0.825 for the patients receiving the acetate-containing dialysate, and $R^2$ was equal to 0.831 for the patients receiving the acetate-free dialysate.

The parameters predicted to contribute greater than a pre-set amount in the post-dialysis patient bicarbonate level can be identified as the significant parameters. The pre-set amount can be determined according to technical and clinical data in the art. For example, the pre-set amount can be $\pm 2.0$ mM, or any other numbers applicable in the art.

The significant parameters can also be identified by criteria not limited to the P-B screen results. For example, the concentrations of calcium and magnesium ions in the cation infusate can be categorized as the significant parameters due to the significance of the concentration of potassium ions in the cation infusate indicated by the P-B screen result, and an arbitrary choice to make potassium more variable than calcium in the screening.

In a non-limiting example, 11 parameters in Table 1 were identified as significant parameters in predicting the post-dialysis patient bicarbonate level by P-B screening. Three of these significant parameters were patient characteristics (patient pre-dialysis bicarbonate level, patient pre-dialysis urea level, and total body water), three were device pump flow rates (dialysate flow rate, blood flow rate, and bicarbonate concentrate flow rate), four were based on the infusate prescription (concentrations of calcium, potassium, and magnesium in the cation concentrate and initial concentration of sodium in the dialysate) and one was a sorbent cartridge property (sorbent cartridge pH level.)

Once identified, the significant parameters can be subject to a two-level full factorial analysis at step **630**. The significant parameters can be set for extreme ranges representing the high and low levels and the other adjustable parameters to their respective center values. The number of runs can be determined by the number of significant factors. For example, the two-level full factorial analysis for the 11 significant parameters was done in $2^{11}$ (2048) runs for each acetate case and each acetate-free case in view of the other 29 adjustable parameters at fixed, nominal values.

A response of post-dialysis patient bicarbonate level can be fit to a linear transfer function (LTF) of main effects and two-way interactions for each case by using any suitable statistical tools, such as MATLAB's stepwise linear fit (*stepwiselm*), at step **640**. A linear fit of the post-dialysis patient bicarbonate level response to main effects and two-way interactions can be done for the patients receiving the acetate-containing dialysate and the patients receiving the acetate-free dialysate. In a non-limiting example, 11 significant parameters were analyzed, $R^2$ in the response that fits the linear model for the patients receiving acetate was 0.982, and the $R^2$ was 0.983 for the patients not receiving acetate.

Response surface methodology (RSM) can also be applied to analyze the simulation data. RSM is a set of advanced design of experiments techniques, which is often used to optimize the response (output variable) influenced by input variables. A functional form of the LTF for a response of the post-dialysis patient bicarbonate level can be obtained from the RSM results, or any other applicable statistical tools known in the art. The function form can be given below, where $T_0$ denotes the intercept term, $T_i$ denotes the coefficient on main effect $i$, and $V_i$ denotes main effect $i$. $T_{ij}$ denotes the coefficient on interactions of the main effect $V_i$ and other variable $V_j$. The functional form can have different coefficients and variables for the patients receiving acetate-free dialysate and the patients receiving acetate-containing dialysate.

$$Cp_{Base}^{End} = T_0 + \sum T_i V_i + \sum T_{ij} V_i V_j \qquad \text{Eq. (20)}$$

The resulting linear fits from the RSM can be solved for the set point of the base pump flow rate, yielding a rational function with a singularity for each acetate case, referred to as "predictor" for state bicarbonate flow rate (Qbase_0) at step **650**.

$$Q_{base}^0 = \frac{Cp_{Base}^{End} - T_0 - \sum_{i \neq Q_{base}^0} \left( T_i V_i \right)}{T_{Q_{base}^0} + \sum T_q V_q} \qquad \text{Eq. (21)}$$

The predictors can contain two different forms for patients receiving acetate-free dialysate and patients receiving acetate-containing dialysate, respectively. Each predictor can be used to directly predict the flow rate of bicarbonate for a user selected post-dialysis patient bicarbonate level. Alternatively, the predictors can be refined to remove the singularity in the denominator, such as those triggered by unphysical total body water maximum.

Additional linear functions may be obtained by removing the singularity of the predictors. To remedy the singularity problem, an everywhere-defined "corrector" linear transfer function can be fit using each predictor and additional simulations at step **660**. A user can specify the post-dialysis patient bicarbonate level for running the additional stimulations. *Qbase_0* can be assigned by the predictor to collect a set of data of the significant parameters for fitting the corrector. For example, the simulation can be run at least 5000 additional times or any other numbers of additional times that the user determines to be suitable. The user selected post-dialysis patient bicarbonate level can be set at 27.0 mM, or any other levels within the physiological range of the patients.

Additional simulation data can be generated by a sampling method, for example, Latin hypercube sampling, which is a statistical method used to generate controlled random samples of uniform distributions in discrete bins between values 0 and 1 which can be mapped to an arbitrary distribution containing the low and high extreme ranges of the significant factors by way of an inverse cumulative distribution function. Latin hypercube sampling can be used to obtain a space-filling dataset for fitting, which spreads the sample points more evenly across all possible values. Other sampling methods may also be used to generate the additional data.

Using the predictor equations and the further simulations, two additional LTFs, referred to as "corrector linear transfer functions," can be fit to a response of base flow rate required to reach a post-dialysis patient bicarbonate level. For example, regarding 11 significant parameters, the corrector for the patients receiving an acetate-containing dialysate had an $R^2$ equal to 0.964, and the corrector for the patients receiving an acetate-free dialysate had an $R^2$ equal to 0.978.

In any embodiment of the first, second, third, fourth, fifth, or sixth aspects of the present disclosure, the corrector functional form can be given as follow:

$$Q_{base}^0 = T_0 + \sum T_i V_i + \sum T_{ij} V_i V_j \qquad \text{Eq. (22)}$$

In Eq. (22), $T_0$ denotes the intercept term, $T_i$ denotes the coefficient on main effect $i$, and $V_i$ denotes main effect $i$. $T_{ij}$ denotes the coefficient on interactions of the main effect $V_i$ and other variable $V_j$. The functional form can have different coefficients and variables for the patients receiving acetate-free dialysate and the patients receiving acetate-containing dialysate. The correctors can have at least one of different coefficients and variables for patients receiving acetate-free dialysate and patients receiving acetate-containing dialysate. Correctors can be used to predict the flow rate of bicarbonate to result in a target post-dialysis patient bicarbonate level, and generally provide more accurate determination of the start base-flow rate, as compared to the predictors, for controlling the post-dialysis patient bicarbonate level within a predetermined range.

The range of the post-dialysis patient bicarbonate level can be significantly reduced when using the flow rate of bicarbonate determined by the personalized control algorithms, as compared to using a universal rate for any member of the patient population. For example, the range of total bicarbonate levels was reduced from 41.0 mM (8.0-49.0 mM) to 21.2 mM (18.9-40.1 mM) using the personalized base-flow rate obtained from a corrector linear transfer function as opposed to using a universal rate on a computer simulated population of patients with extreme-valued patient and system parameters. Standard deviation was also be reduced from 8.7 mM to 4.5 mM (F-test, p = $8.6 \times 10^{-189}$ << 0.05). Once the control algorithms are obtained by curve fitting, the system can apply the control algorithms to set the personalized bicarbonate addition profile.

*Experiment 1*

A sorbent hemodialysis mathematical model based upon the previously described embodiment using a system of ordinary differential equations was programmed with the C++ programming language and was compiled as an executable for running on a mobile device operating on the Android platform. The CVODE library version 2.8.2 for stiff and nonstiff ordinary differential equation systems was used as the numerical methods solver. The treatment parameters in Table 2 were assumed, and the algorithm illustrated in FIG. 3 was executed for targeting a post-dialysis bicarbonate level of 28 mM in the patient, which was determined to correspond to a dialysate bicarbonate concentration of 34.8 mmol/L by an iterative method. A titration profile for the pre-degasser effluent pH of the sorbent cartridge with slope $dpH/dB$ = - 2.8658*exp(-3.66*$B$) + 0.6627*exp(-0.6627*$B$) was assumed, where $B$ represents the cumulative total CO2 mass exposure of the zirconium phosphate layer per unit mass of ZP in units of mol/kg. Table 3 provides a sample of algorithm outputs considering the assumed inputs in Table 2.

Table 2

| Input Label | Input Value |
|---|---|
| Hemodialysis treatment duration | 240 min |
| Hemodialysis treatment ultrafiltration rate(s) and/or profile(s) | Constant 5.0 mL/min |
| Hemodialysis treatment dialysate flow rate(s) and/or profile(s) | Constant 500 mL/min |
| Hemodialysis treatment blood flow rate(s) and/or profile(s) | Constant 300 mL/min |
| Hemodialysis treatment sodium prescription(s) and/or profile(s) | Constant 140.0 mM |
| Hemodialysis treatment infusate prescription(s) and/or profile(s) | Constant values: 1.5 mM calcium, 2.0 mM potassium, 0.375 mM magnesium, 5.5 mM glucose |
| Patient pre-dialysis bicarbonate level(s) | 21.0 mM |

(continued)

| Input Label | Input Value |
|---|---|
| Patient pre-dialysis urea level(s) | 16.4 mM (as urea) |
| Patient pre-dialysis weight(s) and/or total body water volume(s) | 32.0 L |
| Patient acid generation and/or bicarbonate consumption rate(s) | 0.6 mmol/min |
| Sorbent cartridge mass(es) and/or layer order(s) | 2.2 kg (Mass of Zirconium Phosphate) |
| Sorbent cartridge pH level(s) and/or profile(s) | 6.5 (pH minimum of Zirconium Phosphate) |
| Device dialyzer efficiency (K0A) | 1.645 L/min |
| Device degasser outlet PCO2 level(s) and/or profile | Constant 65.0 mmHg |
| Whether bicarbonate is metered by flow-through cartridge of liquid concentrate | Flow-through cartridge |

Table 3

| Time (t) | QAdd(t) |
|---|---|
| 0 min | 3.69 mmol/min |
| 15 min | 4.53 mmol/min |
| 30 min | 5.27 mmol/min |
| 45 min | 5.69 mmol/min |
| 60 min | 5.87 mmol/min |
| 75 min | 5.89 mmol/min |
| 90 min | 5.81 mmol/min |
| 105 min | 5.67 mmol/min |
| 120 min | 5.50 mmol/min |
| 135 min | 5.33 mmol/min |
| 150 min | 5.15 mmol/min |
| 165 min | 4.99 mmol/min |
| 180 min | 4.83 mmol/min |
| 195 min | 4.69 mmol/min |
| 210 min | 4.56 mmol/min |
| 225 min | 4.44 mmol/min |
| 240 min | 4.34 mmol/min |

**Claims**

1. A dialysis system, comprising:

   a dialysate flow path (30), the dialysate flow path comprising a dialyzer (20), dialysate pump (48), and a bicarbonate source (34); and
   a sorbent cartridge receiving spent dialysate from the dialysate flow path for regeneration and return to the dialysate flow path;
   a processor programmed to obtain one or more patient parameters and one or more system parameters wherein the system parameters include sorbent cartridge mass, sorbent cartridge layer order, and sorbent cartridge pH and/or profile and to set a flow rate of bicarbonate from the bicarbonate source to the dialysate flow path for achieving a target patient bicarbonate concentration,

wherein the processor sets the flow rate of bicarbonate from the bicarbonate source to the dialysate flow path based on a user specified patient post-dialysis bicarbonate level,

which user specified patient post-dialysis bicarbonate level corresponds to the target patient bicarbonate concentration and whereby the system is instructed by the user to use this target for setting the bicarbonate flow rate.

2. The dialysis system of claim 1, wherein the system parameters include one or more selected from the group consisting of dialysis treatment duration, ultrafiltration rate and/or ultrafiltration profile, dialysate flow rate and/or profile, blood flow rate and/or profile, sodium prescription and/or profile, infusate prescription and/or profile, dialyzer efficiency, degasser outlet $PCO_2$ levels and/or profile, whether bicarbonate is to be metered by a flow-through cartridge or liquid concentrate, dialysis mode, and dialysate temperature.

3. The dialysis system of claim 1 or 2, wherein the patient parameters include one or more selected from the group consisting of patient pre-dialysis bicarbonate level, patient pre-dialysis urea level, patient pre- dialysis weight, patient acid generation, and patient bicarbonate consumption rate.

4. The dialysis system of any of the preceding claims wherein the processor sets a dialysate bicarbonate prescription or profile based on the user specified patient post-dialysis bicarbonate level by an iterative algorithm, preferably wherein the processor is programmed to operate a mathematical model using the one or more patient parameters and the one or more system parameters to set a personalized bicarbonate addition profile and a set patient bicarbonate concentration.

5. The dialysis system of claim 4, wherein the processor is programmed to operate a mathematical model using the one or more patient parameters and the one or more system parameters to set a personalized bicarbonate addition profile and a set patient bicarbonate concentration, wherein the processor is programmed to determine whether a difference between the user specified patient bicarbonate level and the set patient bicarbonate concentration is greater than a predetermined threshold.

6. The dialysis system of any of the preceding claims wherein at least one patient parameter is obtained from population averages and/or historical values.

7. The dialysis system of any of the preceding claims, wherein the processor is programmed to set the flow rate of bicarbonate using any of:

   a) curve fitting;
   b) partial differential equations;
   c) ordinary differential equations; or
   d) a look up table,

   preferably wherein the curve fitting includes any one or more of:

   1. a linear transfer function; or
   11. a non-linear transfer function.

8. The dialysis system of any of the preceding claims, wherein the processor is programmed to set a personalized bicarbonate addition profile, wherein the personalized bicarbonate addition profile uses steps, linear interpolations, non-linear interpolations, and/or continuous addition.

9. The dialysis system of any of the preceding claims, wherein the processor is programmed to set a second flow rate of bicarbonate during dialysis treatment.

**Patentansprüche**

1. Dialysesystem, umfassend:

   einen Dialysatströmungspfad (30), der Dialysatströmungspfad umfassend einen Dialysator (20), eine Dialysatpumpe (48) und eine Bikarbonatquelle (34); und

eine Sorbenskartusche, die verbrauchtes Dialysat aus dem Dialysatströmungspfad für eine Regeneration und eine Rückführung in den Dialysatströmungspfad aufnimmt;

einen Prozessor, der programmiert ist, um einen oder mehrere Patientenparameter und einen oder mehrere Systemparameter zu erhalten, wobei die Systemparameter eine Sorbenskartuschenmasse, eine Reihenfolge von Schichten der Sorbenskartusche und einen pH-Wert und/oder ein Profil der Sorbenskartusche einschließen, und um eine Strömungsrate von Bikarbonat von der Bikarbonatquelle zu dem Dialysatströmungspfad festzulegen, um eine Zielbikarbonatkonzentration des Patienten zu erreichen,

wobei der Prozessor die Strömungsrate von Bikarbonat von der Bikarbonatquelle zu dem Dialysatströmungspfad basierend auf einem Benutzer-spezifizierten Bikarbonatspiegel eines Patient nach der Dialyse festlegt, der Benutzer-spezifizierte Bikarbonatspiegel des Patienten nach der Dialyse der Zielbikarbonatkonzentration des Patienten entspricht und wodurch das System durch den Benutzer angewiesen wird, diesen Zielwert zum Festlegen der Bikarbonatströmungsrate zu verwenden.

2. Dialysesystem nach Anspruch 1, wobei die Systemparameter einen oder mehrere Parameter einschließen, die aus der Gruppe ausgewählt sind, bestehend aus Dialysebehandlungsdauer, Ultrafiltrationsrate und/oder Ultrafiltrationsprofil, Dialysatströmungsrate und/oder -profil, Blutströmungsrate und/oder -profil, Natriumverordnung und/oder -profil, Infusatverordnung und/oder -profil, Dialysatoreffizienz, PCO2-Spiegel und/oder -Profil eines Entgaserauslasses, ob Bikarbonat durch eine Durchströmungskartusche oder ein flüssiges Konzentrat dosiert werden soll, Dialysemodus und Dialysattemperatur.

3. Dialysesystem nach Anspruch 1 oder 2, wobei die Patientenparameter einen oder mehrere Parameter einschließen, die aus der Gruppe ausgewählt sind, bestehend aus Bikarbonatspiegel des Patienten vor der Dialyse, Harnstoffspiegel des Patienten vor der Dialyse, Gewicht des Patienten vor der Dialyse, Säureproduktion des Patienten und Bikarbonatverbrauchsrate des Patienten.

4. Dialysesystem nach einem der vorstehenden Ansprüche, wobei der Prozessor eine Dialysatbikarbonatverordnung oder ein Dialysatbikarbonatprofil basierend auf dem Benutzer-spezifizierten Bikarbonatspiegel des Patienten nach der Dialyse durch einen iterativen Algorithmus festlegt, vorzugsweise wobei der Prozessor programmiert ist, um ein mathematisches Modell unter Verwendung des einen oder der mehreren Patientenparameter und des einen oder der mehreren Systemparameter zu betreiben, um ein personalisiertes Bikarbonataddtionsprofil und eine festgelegte Patientenbikarbonatkonzentration festzulegen.

5. Dialysesystem nach Anspruch 4, wobei der Prozessor programmiert ist, um unter Verwendung des einen oder der mehreren Patientenparameter und des einen oder der mehreren Systemparameter ein mathematisches Modell zu betreiben, um ein personalisiertes Bikarbonataddtionsprofil und eine festgelegte Patientenbikarbonatkonzentration festzulegen, wobei der Prozessor programmiert ist, um zu bestimmen, ob eine Differenz zwischen dem benutzerdefinierten Patientenbikarbonatspiegel und der festgelegten Patientenbikarbonatkonzentration größer als ein zuvor bestimmter Schwellenwert ist.

6. Dialysesystem nach einem der vorstehenden Ansprüche, wobei mindestens ein Patientenparameter aus Bevölkerungsdurchschnitten und/oder historischen Werten gewonnen wird.

7. Dialysesystem nach einem der vorstehenden Ansprüche, wobei der Prozessor programmiert ist, um die Strömungsrate von Bikarbonat unter Verwendung festzulegen von:

a) Kurvenanpassung;
b) partielle Differentialgleichungen;
c) gewöhnliche Differentialgleichungen; oder
d) eine Nachschlagetabelle,
vorzugsweise wobei die Kurvenanpassung eines oder mehrere einschließt von:

1. einer linearen Übertragungsfunktion; oder
11. einer nichtlinearen Übertragungsfunktion.

8. Dialysesystem nach einem der vorstehenden Ansprüche, wobei der Prozessor programmiert ist, um ein personalisiertes Bicarbonataddtionsprofil festzulegen, wobei das personalisierte Bicarbonataddtionsprofil Schritte, lineare Interpolationen, nichtlineare Interpolationen und/oder kontinuierliche Addition verwendet.

9. Dialysesystem nach einem der vorstehenden Ansprüche, wobei der Prozessor programmiert ist, um während einer Dialysebehandlung eine zweite Strömungsrate von Bikarbonat festzulegen.

**Revendications**

1. Système de dialyse comprenant :

   une voie d'écoulement du dialysat (30), la voie d'écoulement du dialysat comprenant un dialyseur (20), une pompe à dialysat (48) et une source de bicarbonate (34) ; et
   une cartouche de sorbant recevant le dialysat usé de la voie d'écoulement du dialysat pour le régénérer et le renvoyer dans la voie d'écoulement du dialysat ;
   un processeur programmé pour obtenir un ou plusieurs paramètres du patient et un ou plusieurs paramètres du système, dans lequel les paramètres du système comportent la masse de la cartouche de sorbant, l'ordre des couches de la cartouche de sorbant et le pH et/ou le profil de la cartouche de sorbant, et pour régler un débit de bicarbonate de la source de bicarbonate à la voie d'écoulement du dialysat afin d'atteindre une concentration cible de bicarbonate pour le patient,
   dans lequel le processeur règle le débit de bicarbonate de la source de bicarbonate à la voie d'écoulement du dialysat sur la base d'un niveau de bicarbonate post-dialyse du patient spécifié par l'utilisateur,
   lequel niveau de bicarbonate post-dialyse du patient spécifié par l'utilisateur correspond à la concentration cible de bicarbonate du patient et l'utilisateur demande au système d'utiliser cette cible pour régler le débit de bicarbonate.

2. Système de dialyse selon la revendication 1, dans lequel les paramètres du système comportent un ou plusieurs paramètres choisis dans le groupe consistant en la durée du traitement de dialyse, le taux d'ultrafiltration et/ou le profil d'ultrafiltration, le débit et/ou le profil du dialysat, le débit et/ou le profil du sang, la prescription et/ou le profil du sodium, la prescription et/ou le profil du perfusat, l'efficacité du dialyseur, les niveaux et/ou le profil de PC02 à la sortie du dégazeur, si le bicarbonate doit être dosé par une cartouche à écoulement continu ou un concentré liquide, le mode de dialyse, et la température du dialysat.

3. Système de dialyse selon la revendication 1 ou 2, dans lequel les paramètres du patient comportent un ou plusieurs paramètres choisis dans le groupe constitué du niveau de bicarbonate prédialyse du patient, du niveau d'urée prédialyse du patient, du poids prédialyse du patient, de la génération d'acide du patient et du taux de consommation de bicarbonate du patient.

4. Système de dialyse selon l'une quelconque des revendications précédentes, dans lequel le processeur établit une prescription ou un profil de bicarbonate de dialyse basé sur le niveau de bicarbonate post-dialyse du patient spécifié par l'utilisateur par un algorithme itératif, de préférence dans lequel le processeur est programmé pour faire fonctionner un modèle mathématique à l'aide d'un ou de plusieurs paramètres du patient et un ou plusieurs paramètres du système pour établir un profil d'ajout de bicarbonate personnalisé et une concentration de bicarbonate du patient définie.

5. Système de dialyse selon la revendication 4, dans lequel le processeur est programmé pour faire fonctionner un modèle mathématique à l'aide d'un ou de plusieurs paramètres du patient et un ou plusieurs paramètres du système pour définir un profil d'ajout de bicarbonate personnalisé et une concentration de bicarbonate du patient, dans lequel le processeur est programmé pour déterminer si une différence entre le niveau de bicarbonate du patient spécifié par l'utilisateur et la concentration de bicarbonate du patient définie est supérieure à un seuil prédéterminé.

6. Système de dialyse selon l'une quelconque des revendications précédentes, dans lequel au moins un paramètre patient est obtenu à partir de moyennes de population et/ou de valeurs historiques.

7. Système de dialyse selon l'une quelconque des revendications précédentes, dans lequel le processeur est programmé pour régler le débit de bicarbonate en utilisant l'un des éléments suivants :

   a) l'ajustement des courbes ;
   b) les équations différentielles partielles ;
   c) les équations différentielles ordinaires ; ou
   d) une table de recherche,

de préférence, dans lequel l'ajustement de la courbe comporte un ou plusieurs des éléments suivants :

       1. une fonction de transfert linéaire ; ou
      11. une fonction de transfert non linéaire.

**8.** Système de dialyse selon l'une quelconque des revendications précédentes, dans lequel le processeur est programmé pour définir un profil d'ajout de bicarbonate personnalisé, dans lequel le profil d'ajout de bicarbonate personnalisé utilise des étapes, des interpolations linéaires, des interpolations non linéaires, et/ou un ajout continu.

**9.** Système de dialyse selon l'une quelconque des revendications précédentes, dans lequel le processeur est programmé pour régler un second débit de bicarbonate pendant le traitement par dialyse.

FIG. 1

FIG. 2

204

Algorithm Determines Dialysate Bicarbonate Rx to Achieve Treatment Target(s) in Patient

210

Re-run Bicarbonate Algorithm Considering Treatment History and Updated Parameter(s)

211

Resume Treatment Using New Metering Profile Output from Algorithm

201

Start: Enter Parameters
- Treatment
- Patient
- Sorbent
- Device
- Other Parameters

202

Target Bicarbonate in Patient?

Yes

No

205

Run Mathematical Model with Dialysate Bicarbonate Rx

206

Metering Profile to Achieve Intended Dialysate Bicarbonate Rx Available for Use

207

Begin Treatment with Bicarbonate Metering Profile Output from Mathematical Model

208

Use Case Requiring Recalculation Occurs?

Yes

No

203

User Specifies Dialysate Bicarbonate Rx

209

Finish Treatment with Bicarbonate Metering Profile Output from Algorithm

212

End:
- Treatment Bicarbonate Rx
- Post-HD Bicarbonate

FIG. 3

Hemodialsyis session     Bicarbonate pump flow rate

Start          End

a. constant rate

b. linear change

c. different rates

d. other combinations

Total mass of bicarbonate delivered

FIG. 4

FIG. 5

610 | Data simulation of parameters

620 | P-B Screen to determine significant parameters from the adjustable parameters

Adjustable parameters set for extreme ranges

630 | Full factorial analysis on the determined significant parameters

Significant parameters set for extreme ranges (main effects)
Others set for center values (potential interactions)

640 | Obtaining Linear Transfer Function (LTF) of the total-base level response with two-way interactions

Solve LTF of total base level for start base-flow rate

650 | Obtaining LTF predictor algorithms for start base-flow rate

Additional data simulations based on the predictor algorithm
Remove singularity from the predictor using the additional data

660 | Obtaining LTF corrector algorithms for start base-flow rate

FIG. 6

REFERENCES CITED IN THE DESCRIPTION

**Patent documents cited in the description**

- US 2015343126 A1
- US 2013213890 A1
- US 2014158588 A1